Europäisches Patentamt

European Patent Office

Office européen des brevets

(11) Publication number: **0 061 430**
**B1**

(12) **EUROPEAN PATENT SPECIFICATION**

(45) Date of publication of patent specification: **10.09.86**

(21) Application number: **82830035.0**

(22) Date of filing: **19.02.82**

(51) Int. Cl.⁴: **A 61 F 2/24**

(54) Cardiac valvular prosthesis of mechanical type provided with two freely moving leaflets.

(30) Priority: **17.03.81 IT 4804081**
**11.12.81 IT 4989381**

(43) Date of publication of application:
**29.09.82 Bulletin 82/39**

(45) Publication of the grant of the patent:
**10.09.86 Bulletin 86/37**

(84) Designated Contracting States:
**AT BE CH DE FR GB LI LU NL SE**

(56) References cited:
**EP-A-0 023 797**
**FR-A-2 331 997**
**GB-A-1 160 008**
**GB-A-1 167 329**
**US-A-3 903 548**
**US-A-4 078 268**
**US-A-4 183 103**
**US-A-4 240 161**

(73) Proprietor: **Marconi, Valter**
**Via A. Barilatti n. 72**
**I-00144 Roma (IT)**

(72) Inventor: **Marconi, Valter**
**Via A. Barilatti n. 72**
**I-00144 Roma (IT)**

Courier Press, Leamington Spa, England.

## Description

This invention relates to an artificial cardiac valve of mechanical type, provided with two leaflets mobile with a rotation-translation movement.

A good prosthesis must possess many requirements, concerning both fabrication material and valve design.

Good results, from the hemodynamic point of view, were obtained by valves such as the "Edinburgh valve" by McLeod, or the "St. Jude Medical valve", where the occluder, respectively consisting of a disc or of two semi-circular flat leaves, puts itself, in the position of maximum opening, almost parallel to the hematic flow lines.

A remarkable disadvantage of this type of valves is the presence of hinges, necessary in order to permit the fast rotation of the occluder. These hinges proved to be dangerous starting points for the formation of clots (L. Nunez et al. Annals Thoracic Surgery, 29, No. 6, 567 (1980).

Another class of valves is described in US—A—3,903,548, where the occluder consists of two freely floating discs. In this realization however, in order to occupy and cover completely (in the closure position) the circular surface area of the base ring by two non hinged and freely floating discs, it is necessary not withstanding the inclination of the disc acting as occluder, to introduce fixed elements of occlusion that decrease the EOA and consequently the flow, and can cause thrombogenicity, due to the non-laminarity of the blood flow.

The last category of valves includes US—A—4,078,268. This bileaflet valve is characterized in that the opening of the leaflets is "reverse" with respect to the flow direction (with remarkable flow turbulence), and the rotational axes are very far from the diametral edges of the leaflets. In other words, the rotation fulcra are chords of a circle and are very distant from the central diameter of the circle.

This structure gives rise, in the open position, to two lateral flow chambers having a small lumen, with consequent thrombogenesis problems.

From the previously described state of the art it turns out that a valve of new type, possessing a low profile occluder (able to guarantee flow laminarity and maximum orifice area), and requiring no hinges, joints or similar to ensure its correct motion, would represent a real progress in the exploitation of prosthetic cardiac valves.

The present invention precisely relates to a new cardiac valvular prosthesis, of mechanical type, consisting of:

1) an external sewing ring

2) a housing ring forming a flow orifice

3) two semi-circular or semi-ellipsoidal leaflets opening and closing the valve orifice by pivoting about fulcra and sliding over diametrically opposed slipping surfaces provided on the structure of the housing ring itself

4) a constraint structure aimed at retaining the leaflets in the housing ring.

This structure of metallic elements, for example wire, generally consists of a part close to the leaflets concavity (if the leaflet has a curvature) and acting as rotation fulcrum, and of one or more arches or arch segments (per each leaflet) having the function of guaranteeing the guidance and the correct positioning of the leaflet (constraint structure).

From the veiwpoint of manufacture, these structures can be separated and independent, as well as they can form part of one structure devoid of discontinuity.

The "rotation structure", i.e. that part of the system of metallic elements having the function of permitting the leaflets to rotate, may consist of short metal filaments, often provided with a slight curvature, and welded to the flow orifice of the valve.

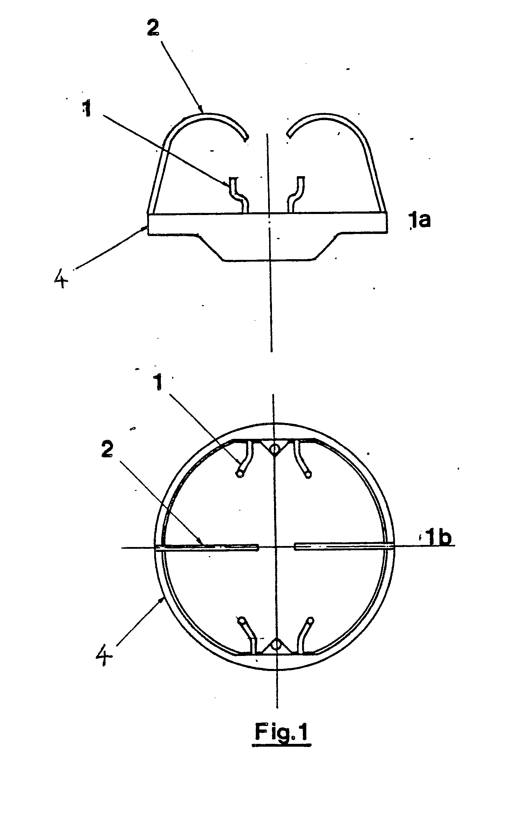

Figure 1a shows a side view of a valve where 1 is the rotation fulcrum of the leaflet (not represented in the drawing for sake of simplicity), 2 represents the guide and constraint structure of the valve, 4 represents the flow orifice (valve housing).

Fig. 1b represents the plan view of the same embodiment.

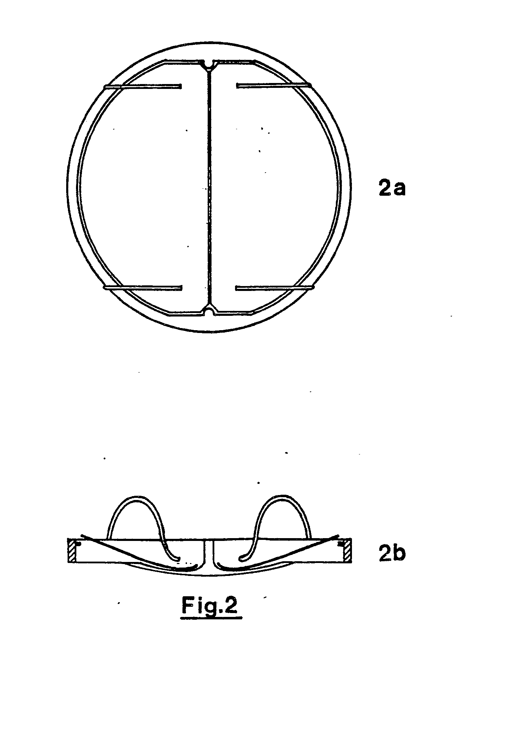

Fig. 2a shows the plan view of another embodiment where the rotation fulcrum of the valve and the guide and positioning structure are realized by means of one metallic element.

Fig. 2b shows the side view of the same embodiment.

Figures 3a and 3b show an embodiment in which the constraint structure 2 consists of short wire welded over the housing ring of the valve to form four curved eyelets, whereas in Figures 3c and 3d the constraint structure 2 consists of wire forming only two curved eyelets.

In Figure 3c and 3d there is also shown (reference number 5), the sewing ring of the valve.

Fig. 4a shows a semi-ellipsoidal leaflet where the half-length of the base edge BC is shorter than the segment AC.

Figures 4b and 4c show the edges in relief (ref. no. 10), located in the internal part of the flow orifice and perpendicular to the housing plane of the valve (i.e. parallel to the flow lines across the orifice).

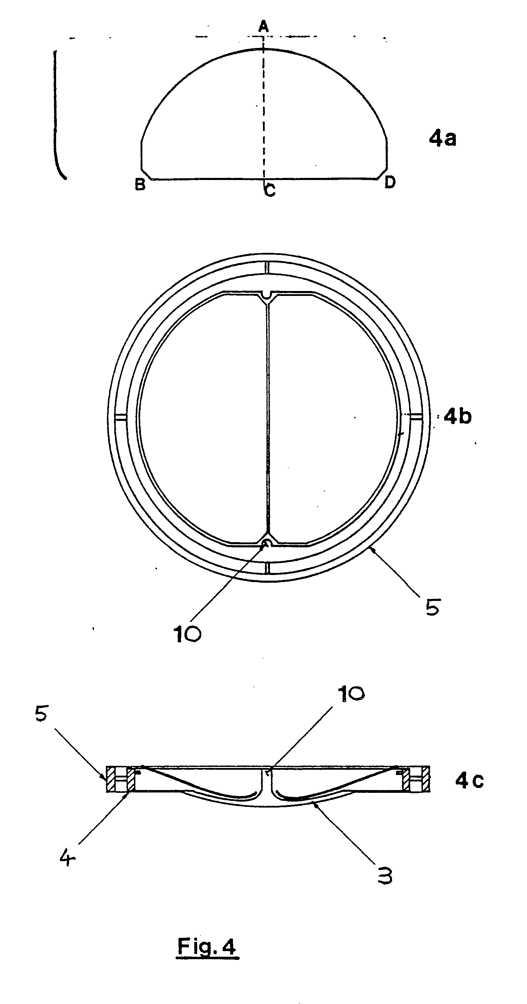

In Figures 4b and 4c there is also indicated, with ref. no. 5, the sewing ring of the valve.

Fig. 4c shows a typical embodiment of the slipping surface 3, inside the ring 4.

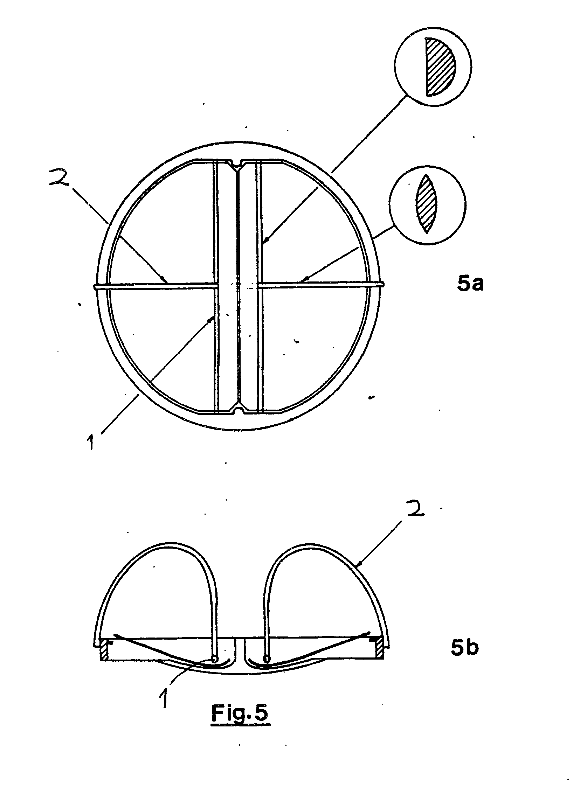

Fig. 5 shows a valve where rotation fulcra and constraint structures are realized by means of one metallic structure, in a continuous way; moreover, in this realization, the section of the constraint structure 2 is "wedge-like", whereas the section of the rotation structure 1 is semi-circular.

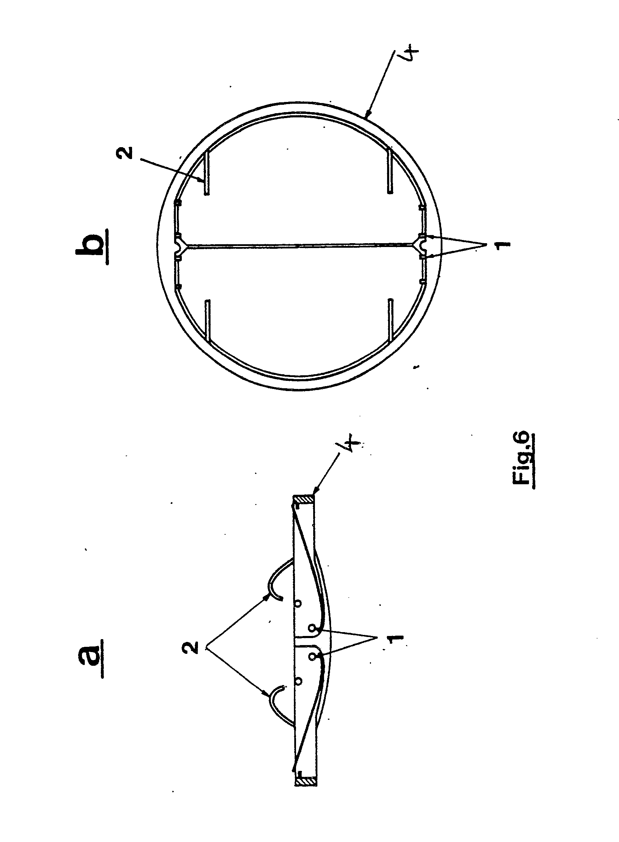

Fig. 6 shows two views of an embodiment where the rotation fulcra of the valve are formed by the pins 1; 2 represents the constraint structures of the valve, 4 represents the flow orifice ring.

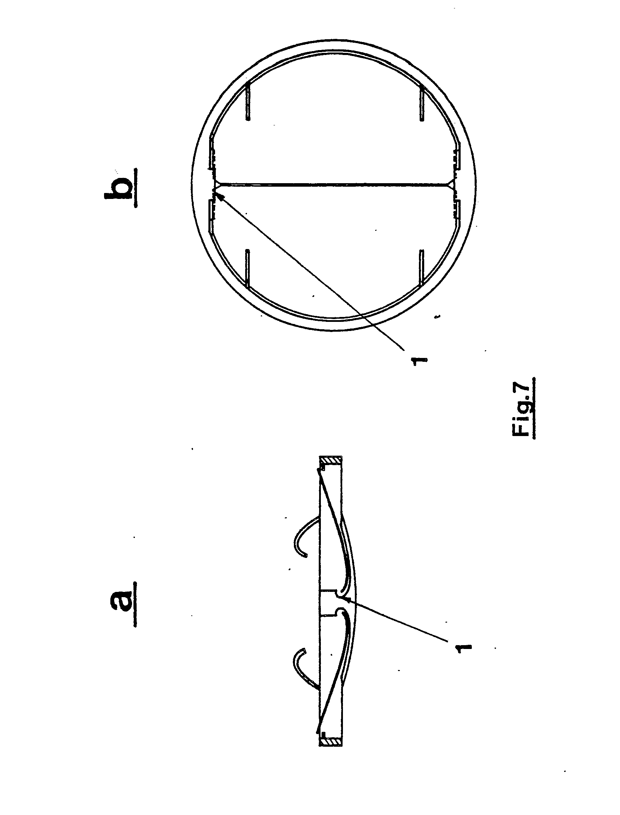

Figure 7 shows two views of a valve where the rotation fulcrum structure 1 is made out of the body itself of the flow orifice ring of the valve.

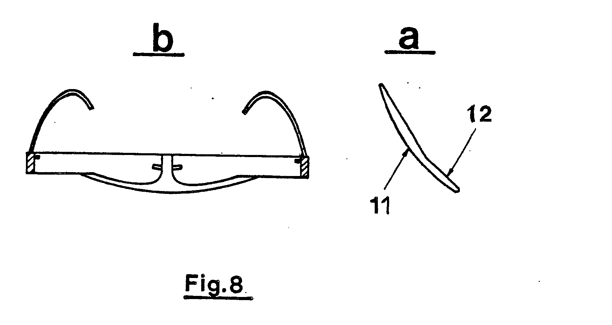

Fig. 8 shows an embodiment where the leaflet sides consist of a convex surface (side 11) and of two plane surfaces forming an angle between each other (side with ref. no. 12).

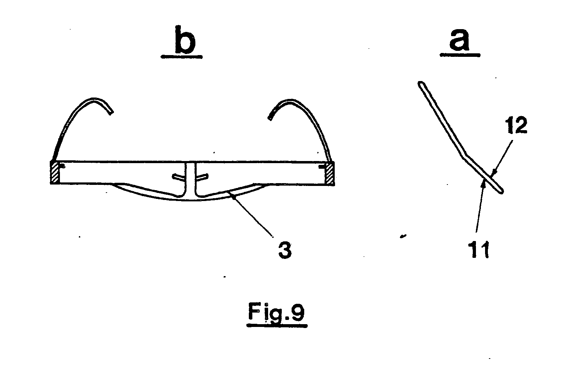

In Fig. 9 each of the sides (11 and 12) of the leaflets consists of two plane surfaces forming an angle between each other. For this reason, the profile of the slipping surface 3 is straight instead of curved.

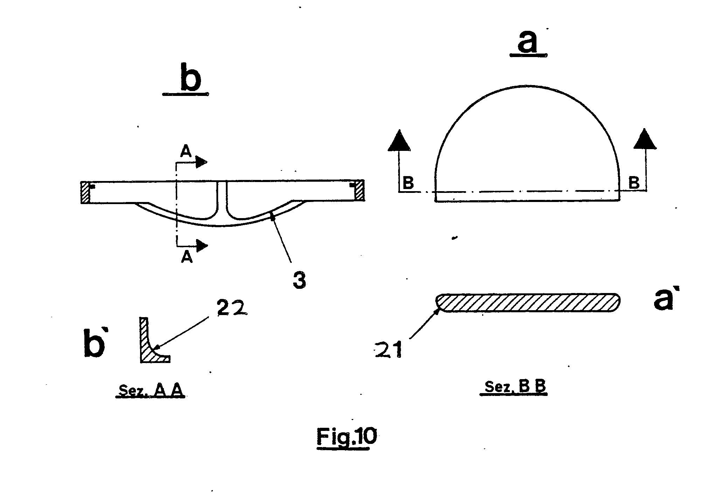

Fig. 10 shows a valve where the side edge 21 of the leaflet has a rounded profile (Fig. 10 and 10a'), whose convexity is turned towards the valve slipping surface 3, whose section 22 is curved, with the concavity turned toward the edge of the leaflet (Fig. 10b and 10b').

Furthermore, Fig. 9b and 10b also illustrate slipping surfaces 3 (both straight and curved) having an ascending profile, with a Z-coordinate (with respect to the basis plane of the valve) higher at the end of the surface than in the middle of the valve.

The rotation structure can also be carried out by means of a simple pin, placed just above the leaflet, in such a position that the leaflet is compelled to a rotation-translation movement. A second pin having a double function of guidance and stop, can be placed in an upper position (see Fig. no. 6a). The "rotation fulcrum" structure can also be made out of the body itself of the flow orifice of the valve, and consist of a portion of increased thickness provided in the middle of the valve, above the surfaces supporting the leaflets and letting them slip, as illustrated in Fig. no. 7.

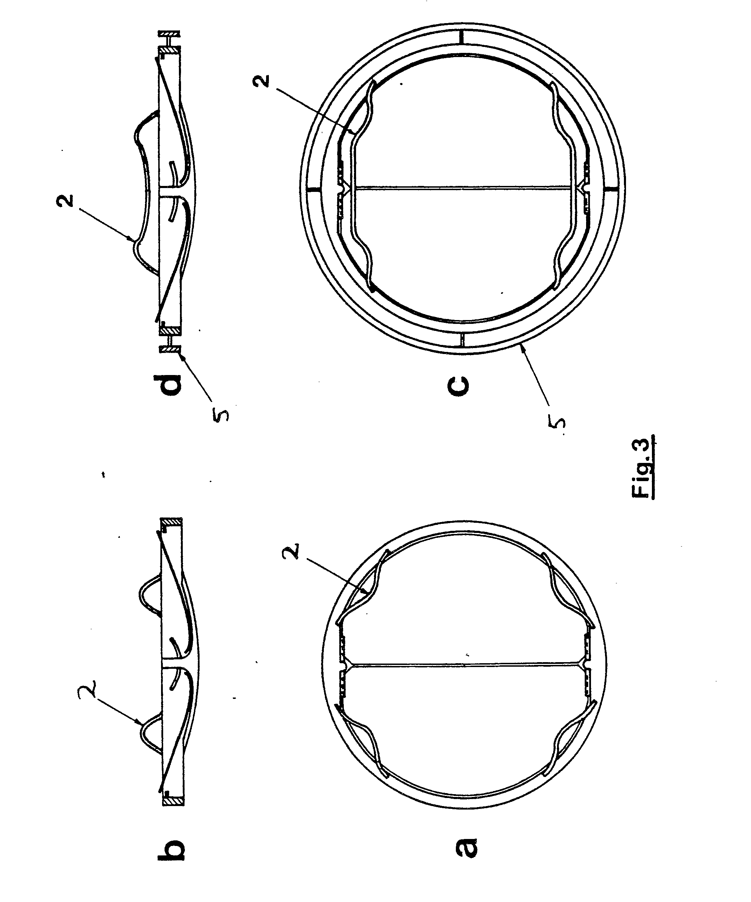

The constraint structure can as well be realized, rather than by means of one or more arches or arch segments, by means of short lengths of wire welded over the housing ring of the valve, to form four or alternatively two curved eyelets, able to prevent the leaflet from getting out, and able to achieve this with minimum flow disturbance (see Fig. 3).

In the opening phase, the leaflets reach quickly a position almost perpendicular to the flow orifice of the valve; this gives rise to two sufficiently broad flow side-sections, which form, together with the central flow section, a system of three openings having a flow section broad enough to guarantee a good flow laminarity.

The rotation-translation movement of the leaflets takes place through slipping of the extremities of the basis edge of the leaflet along a generally curved surface, made out in the inside of the housing ring of the valve, in its central position (this detail appears in almost all figures and particularly Fig. 4 ref. no. 3, Fig. 9 ref. no. 3 and Fig. 10b ref. no. 3).

The valve leaflets, besides semi-circular, can be semi-ellipsoidal, and in this case they show a distance between the apex and the base edge of the leaflet that is higher than the half-length of the base edge (see for a better understanding Fig. 4a, where the half-length of the base edge BC is shown shorter than the distance AC between the base edge BD and the apex A of the leaflet).

This particular geometry makes easier the free slipping of the edge of the leaflet along the surfaces of the housing ring during the opening and closing phases.

In order to make the movement easier and quicker, the leaflets can be provided with a suitable curvature, whose concavity is turned towards the rotation fulcrum of the leaflet (see Fig. 3b and 3d, 4c, 5b, 6a, 7a). The curvature is often located only close to the base edge of the leaflet, whose profile then continues straight on.

The leaflet thickness can be uniform, with both sides (wherever curved) having the same curvature radius, or they can have different curvature radii. The leaflet can so have an uniformly increasing thickness, as well as can be thicker only in some points, as in the middle or near edges.

Alternatively, the leaflet can have the side supported on the slipping surfaces provided with the usual convex profile, whereas the side turned towards the rotation fulcra, instead of being concave, consists of two plane surfaces forming an angle between each other (see Fig. no. 8a). A further improvement of the hemodynamic flow can be achieved by realizing each profile of the leaflet side by two plane surfaces forming an angle between each other, as shown in Fig. 9a.

In another modality of realization of the leaflets, in order to make easier in the phases both of opening and closing of the leaflet its movement along the slipping surfaces, the side edge of the leaflet (or at least the part of the edge being in contact with the surface) has a rounded profile, whose convexity is turned towards the surface. This appears in the Fig. no. 10a', where the Sec. BB shows the abovementioned leaflet section. Since the leaflet structure influences that of the surface, when leaflets having a rounded profile are employed, the section of the surface will also have to be rounded, with a curvature similar to that of the edge of the leaflet, and concavity turned towards it, as shown in the Fig. no. 10b', where the Sec. AA is the surface section.

Similarly, when the leaflet sides consist of plane surfaces forming an angle between each other, also the profile of the surface on which the leaflet slips can be straight instead of curved (see Fig. no. 9b).

In order to decrease even more the opening and closing times of the valve, the slipping surfaces (both curved and straight) can have an ascending profile, and their Z-coordinate with respect to the basis plane of the valve can so be higher at the end of the surface (i.e. towards the apex of the leaflet) than at the origin point of the surface (i.e. in the middle of the housing ring of the valve). This is shown, respectively for a straight or a curved surface, in the Figures nos. 9 and 10 (ref. number 3).

In order to prevent faulty positioning, or even coming out of the leaflets, the flow orifice of the valve can be provided, in its middle, with edges in relief which contact the extremities of the edges of the leaflets in their closure position.

These edges in relief are perpendicular to the flow orifice, are located in the internal part of the flow orifice, and extend from the beginning of the slipping surfaces up to the upper rim of the flow orifice as shown in Fig. 4c (references no. 10).

In the closure position, the leaflets rely on the inside of the valve flow orifice, generally in a slightly inclined position.

The section of the metallic elements forming both the fulcra and the constraint structures can be different from the circular one; rectangular, semi-circular, drop-shaped, or wedge-shaped sections can effectively contribute to improve the hemodynamic flow through the valve.

**Claims**

1. A cardiac valvular prosthesis of mechanical type comprising:
   a) an external sewing ring (5);
   b) a housing ring (4) forming a flow orifice;
   c) two semi-circular or semi-ellipsoidal leaflets which pivot about fulcra (1) for opening and closing the valve orifice, and a constraint structure (2) for retaining the leaflets in the housing ring; characterised in that the leaflets, during opening and closing, both slide over diametrically opposed slipping surfaces (3) provided on the structure of the housing ring itself, said slipping surfaces interacting with the diametral edges of the leaflets, and the fulcra being close to the diametral edges of the leaflets, the structure being such that the leaflets undergo a rotational and translational movement during opening and closing.

2. Valvular prosthesis according to Claim 1, characterised in that the slipping surfaces extend along the internal part of the housing ring.

3. Valvular prosthesis according to Claim 1 or 2, characterised in that the fulcra are provided by curved metal filaments.

4. A mechanical cardiac valve prosthesis as defined in claim 1 or 2, characterized in that each rotation fulcrum consists of two pins on the inside of said housing ring positioned above the leaflet.

5. Valvular prosthesis according to any of the preceding claims characterised in that the valve leaflets are curved close to the diametral edges thereof so as the present a concave face towards the rotation fulcra.

6. Valvular prosthesis according to any of the preceding claims, characterised in that the housing ring has a circular section and the leaflets are semi-ellipsoidal.

**Patentansprüche**

1. Eine mechanische Herzklappenprothese bestehende aus:
   a) einem äusseren Suturring
   b) eine Gehäusering der eine Flussöffnung bildet
   c) zwei halbkreisförmigen oder halb-ellipsenähnlichen Klappen die um Drehpunkte sich drehen um die Oeffnung der Prothese aufzumachen und schliessen, und einer Bindungstruktur um die Klappen im Gehäusering aufzuhalten; dadurch gekennzeichnet, dass beide Klappen, während der Eröffnung und der Schliessung, über genau entgegengesetzte Gleitungsoberflächen, in der eigenen Struktur des Gehäuserings erhaltene, gleiten, diese Gleitungsoberflächen einwirkend auf die diametralen Ränder der Klappen, und wesend die Drehpunkte in der Nähe von den diametralen Rändern der Klappen, wesend die Struktur so dass die Klappen, während der Eröffnung und der Schliessung, mit Rotations un Translationbewegung sich bewegen.

2. Klappenprothese nach Anspruch 1, dadurch gekennzeichnet, dass die Gleitungsoberflächen längs des inneren Teils des Gehäuserings sich erstrecken.

3. Klappenprothese nach Ausprüchen 1 oder 2, dadurch gekennzeichnet, dass dei Rotationsdrehpunkte aus krummen Metallfäden bestehen.

4. Herzklappenprothese nach Ausprüchen 1 oder 2, dadurch gekennzeichnet, dass jeder Rotationsdrehpunkt aus zwei Ueberständen, die innerhalb des obergenannten Gehäuserings, über die Klappe, sich befinden.

5. Klappenprothese nach beliebigen vorherigen Ausprüchen, dadurch gekennzeichnet, dass die Klappen der Klappenprothese in der Nähe ihres diametralen Randes krumm sind, so dass sie eine konkave Seite gegen die Rotationsdrehpunkte zeigen.

6. Klappenprothese nach beliebigen vorherigen Ausprüchen, dadurch gekennzeichnet, dass der Gehäusering einen kreisförmigen Schnitt hat, und die Klappen halb-ellipsenähnlich sind.

**Revendications**

1. Une prothèse valvulaire cardiaque du type mécanique comprenante:
   a) un anneau de suture exterieur
   b) un anneau de logement qui forme un orifice de flux
   c) deux clapets demi-circulaires ou demi-ellipsoïdaux qui ouvrent et ferment l'orifice de la valve en tournant autour de points d'appui pour la rotation, et une structure d'engagement ayante le but d'empécher que les clapets sortent de l'anneau de la valve; caractérisée en ce que lesdits clapets, pendant leur ouverture et fermenture, glissent tous les deux sur des surfaces de roulement diamétralement opposées, situées dans la structure de l'anneau de base même, lesdites surfaces de roulement coopérant avec les bords diamétraux des clapets, et les points d'appui pour la rotation étant placés pres des bords diametraux des clapets, étant la structure telle que les clapets, dans les phases d'ouverture et de fermeture, font un mouvement de rotation et de translation.

2. Prothèse valvulaire selon la revendication 1, caractérisée en ce que les surfaces de roulement se logent le long de la partie intérieure de l'anneau de logement de la valve.

3. Prothèse valvulaire selon les revendications 1 ou 2, caractérisée en ce que les points d'appui pou la rotation consistent dans des filaments métalliques courbes.

4. Une prothèse valvulaire cardiaque du type mécanique, selon les revendications 1 ou 2 caractérisée en ce que chaque structure de points d'appui pour la rotation consiste dans deux protubérances placées dans la partie intérieure de l'anneau de logement, au dessus du clapet.

5. Prothèse valvulaire selon une des revendications précédentes, caractérisée en ce que les clapets de la valve possèdent une courbure près de ses bords diamétraux, de manière qu'ils présentent une face concave vers les points d'appui de la rotation.

6. Prothèse valvulaire selon une des revendications précédentes caractérisée en ce que l'anneau de logement a une section circulaire et les clapets sout demi-ellipsoïdaux.

**Fig.1**

2a

2b

**Fig.2**

**Fig. 3**

0 061 430

Fig. 4

5a

5b

**Fig. 5**

**a**

**b**

2

1

4

2

4

1

Fig.6

0 061 430

0 061 430

## a

## b

**1**

**1**

Fig.7

b

a

12

11

Fig.8

**b**

**a**

12

11

3

**Fig.9**

**Fig.10**